# EUROPEAN PATENT APPLICATION

(11) **EP 4 382 030 A1**
(43) Date of publication of application: **12.06.2024**
(21) Application number: 22306805.7
(22) Date of filing: 07.12.2022
(51) Int. Cl.: A61B 3/028, G02B 27/01, G02C 7/02, G02C 9/04, G02C 13/00

(54) **METHOD AND SYSTEM FOR DETERMINING A PERSONALIZED VALUE OF AN OPTICAL FEATURE OF A CORRECTIVE OPHTHALMIC LENS**

(71) Applicant: Essilor International, 94220 Charenton-Le-Pont (FR)
(72) Inventor: AHANGAMA, Dinesh, 94000 CRETEIL (FR); LE CAIN, Aurelie, 94000 CRETEIL (FR); MARIN, Gildas, 75012 PARIS (FR); TATUR, Guillaume, 77144 MONTEVRAIN (FR)
(74) Representative: Jacobacci Coralis Harle

(57) **Abstract**

The invention relates to a system for determining a personalized value of an optical feature of a corrective ophthalmic lens which is intended to be worn in front of an eye (3) of an individual (2) for correcting his vision, the system comprising:
- a trial optical corrective device (20) adapted to be place in front of an eye of an individual, the trial optical corrective device providing a correction to an individual complying with an initial value of an optical feature;
- an eyewear (30) adapted to be placed in front of the trial optical corrective device worn by the individual and adapted to immerse the individual in a virtual environment, the eyewear being designed to present, in the virtual environment, a visual task to the individual adapted to determine an adjustment value;
- a processing unit programmed calculate a personalized value of said optical feature based at least on the initial value and the adjustment value.

## Description

### TECHNICAL FIELD OF THE INVENTION

The invention relates generally to the field of ophthalmic lenses.

The invention relates more particularly to a method for determining a personalized value of an optical feature of a corrective ophthalmic lens.

### BACKGROUND INFORMATION AND PRIOR ART

Usually, the determination of a value of an optical feature of an ophthalmic lens (for instance a refraction value such as a sphere power), which is personalized for an individual, namely the future wearer of the lens, so as to correct his vision, comprises two phases.

In a first phase, the individual performs a subjective test under the supervision of an eye-care professional, such as an optometrist. From this subjective test, is deduced an initial value of the optical feature.

In a second phase, also called subjective validation phase, the individual is equipped with a trial optical corrective device having the initial value of the optical feature. In other words, this trial optical corrective device reproduces the visual correction determined during the first phase.

Wearing the trial optical corrective device, the individual then performs visual tasks such as reading or looking at targets in far vision. Based on his experience of the visual tasks, the individual may then indicate that the correction provided to him by trial optical corrective device is adopted or complains that the correction is not adapted.

The eye-care professional takes into account the experience of the individual to either validate that the provided correction, that is to say the initial value, suits the individual or to further adjust the initial value.

The second phase is performed in a room, typically in a refraction room or at an optician shop, where space is limited and the illumination is uncontrolled. However, it has for instance been shown that the refraction of the eye, and therefore the correction needed, depends on the spectrum and the intensity of the light as well as gaze direction and object proximity. Moreover, the individual may be poorly installed in the room and stressed out by the presence of the eye-care professional. During the second phase, the individual therefore often adopts non-natural postures.

As a result, the test conditions of the second phase may skew the experience of the individual during the visual tasks. In consequence, the decision taken by the eye-care professional, to either validate or adjust the initial value, may also be skewed.

### SUMMARY OF THE INVENTION

In this context, the invention proposes a method for determining a personalized value of an optical feature of a corrective ophthalmic lens which is intended to be worn in front of an eye of an individual for correcting his vision, the method comprising the steps of:
- determining an initial value of the optical feature based on a visual test performed by the individual;
- equipping the individual with a trial optical corrective device in front of the eye of the individual, the trial optical corrective device providing a correction complying with the initial value of said optical feature and with an eyewear, the trial optical corrective device being placed between the eye and the eyewear, the eyewear being adapted to immerse the individual in a virtual environment;
- determining an adjustment value of said optical feature based on a visual task performed by the individual in the virtual environment;
- calculating the personalized value of said optical feature based at least on the initial value and the adjustment value.

Thanks to the virtual environment, realistic conditions are reproduced for the validation phase. Indeed, in the virtual environment, light conditions or object distances may be controlled. In addition, the field of view seen by the individual in the headset follows the posture and the displacement of the individual. Therefore, the individual adopts more natural postures when performing the visual tasks.

Since the individual is immersed in the virtual environment, he is not disturbed by the presence of the eye-care professional or by the fact that he is in a refraction room or at an optician shop. His experience is therefore closer to his future use of the ophthalmic lens. The virtual environment for instance allows displaying outdoor scenes, which is not possible in the refraction room.

Moreover, thanks to the adaptability of the virtual environment, the visual tasks performed by the individual can reproduce real-life activities of the individual such as car driving or moving around his place. The test conditions of the validation phase are thus extremely relevant to the intended use of the ophthalmic lens.

Thanks to the virtual environment, the visual tasks available for the validation phase are also much more numerous than those available in a refraction room or at an optician shop.

In brief, thanks to the headset, the individual is immersed in a virtual environment which closely reproduces the natural activities and environments in which the ophthalmic lens will be used by the individual. The individual is therefore more likely to have a natural posture and a natural behavior during the validation phase, which strengthen the validation of the initial value or make its adjustment more accurate.

Other advantageous and non-limiting features of the method according to the invention are :
- the trial optical corrective device comprises means adapted to mount the trial optical corrective device in the eyewear, or a frame adapted to be placed on the head of the individual before placing the eyewear on head of the individual;
- the adjustment value is determined based on a subjective assessment of the visual task by the individual or on an objective assessment comprising at least one of the following: a measurement of a posture of the individual, a measurement of a head or gaze direction, a performance or a success rate of the individual performing the visual task;
- the individual is placed in a natural posture when performing the visual task;
- the visual task is representative of at least one of: an activity that the individual intents to perform when wearing the corrective ophthalmic lens, an environment known by the individual, a lifestyle of the individual, an age individual, an environment in which the ophthalmic lens is intended to be worn, preferences of the individual;
- the virtual environment seen by the individual when equipped with the eyewear depends on a position of the head of the individual;
- the virtual environment is generated by virtual reality or mixed reality;
- determining the adjustment value of said optical feature is based on a plurality of visual tasks performed by the individual in the virtual environment;
- the eyewear has at least one of the following parameters: a pixel angle resolution smaller than 1 minute, a projection distance comprised between 75 cm and 1,5 m, a field of view greater than 90 degrees, a display frame rate greater than 60Hz, a luminance greater than 25cd/m²;
- the method further comprises a step of adapting the trial optical corrective device such that the trial optical corrective device provides a correction complying a sum of the initial value and the adjustment value and determining another adjustment value of said optical feature based on another visual task performed by the individual in the virtual environment, and wherein calculating the personalized value of said optical feature is also based on said other adjustment value;
- the method further comprises a step of determining a bias value of the optical feature based on a bias visual task performed by the individual in the virtual environment, said bias visual task being designed such that it reproduces the visual test performed for determining the initial value, and wherein the personalized value is corrected to account for the bias value;
- the method further comprises a step of placing a final optical device in front of the eye of the individual, the final optical device providing a correction complying with the personalized value, and of validating the final optical device based on a validation visual task performed by the individual in the virtual environment;
- the trial optical corrective device comprises at least one of the following: a trial lens having at least one predetermined optical property, an active lens having at least one adaptive optical property, a light field display, a movable lens mounted in a frame such that the distance between the movable ophthalmic lens and the eye is adaptable;
- the method further comprises a step of manufacturing, ordering, or selecting the ophthalmic lens such that the optical feature of the ophthalmic lens is equal to personalized value or to a rounded value determined by rounding the personalized value to the closest or the second closest multiple of a basic value;
- the optical feature comprises at least one of the following: a sphere power; a cylinder power; a cylinder axis; a spherical power addition; a prism power; a prism axis;
- the subjective refraction test comprises at least one of the following: displaying targets placed in a red and green environment; displaying two targets of different sizes; placing, successively, two trial lenses of distinct sphere power in front of the eye; placing, successively, two trial lenses of distinct cylinder power or cylinder axis in front of the eye;

The invention also proposes a system for determining a personalized value of an optical feature of a corrective ophthalmic lens which is intended to be worn in front of an eye of an individual for correcting his vision, the system comprising:
- a trial optical corrective device adapted to be place in front of an eye of an individual, the trial optical corrective device providing a correction to an individual complying with an initial value of an optical feature;
- an eyewear adapted to be placed in front of the trial optical corrective device worn by the individual and adapted to immerse the individual in a virtual environment, the eyewear being designed to present, in the virtual environment, a visual task to the individual adapted to determine an adjustment value;
- a processing unit programmed calculate a personalized value of said optical feature based at least on the initial value and the adjustment value.

### DETAILED DESCRIPTION OF EXAMPLE(S)

The following description, enriched with joint drawings that should be taken as non-limitative examples, will help understand the invention and figure out how it can be realized.

On the appended drawings:
- figure1 is a schematic sectional view from above of a system according to an embodiment of the invention with respect to the eyes of an individual;
- figure 2 is a schematic sectional side-view of the system of the figure 1 ;
- figure 3 is a schematic in perspective of a system according to another embodiment of the invention ;
- figure 4 is a block diagram representing the steps of a method according to the invention.

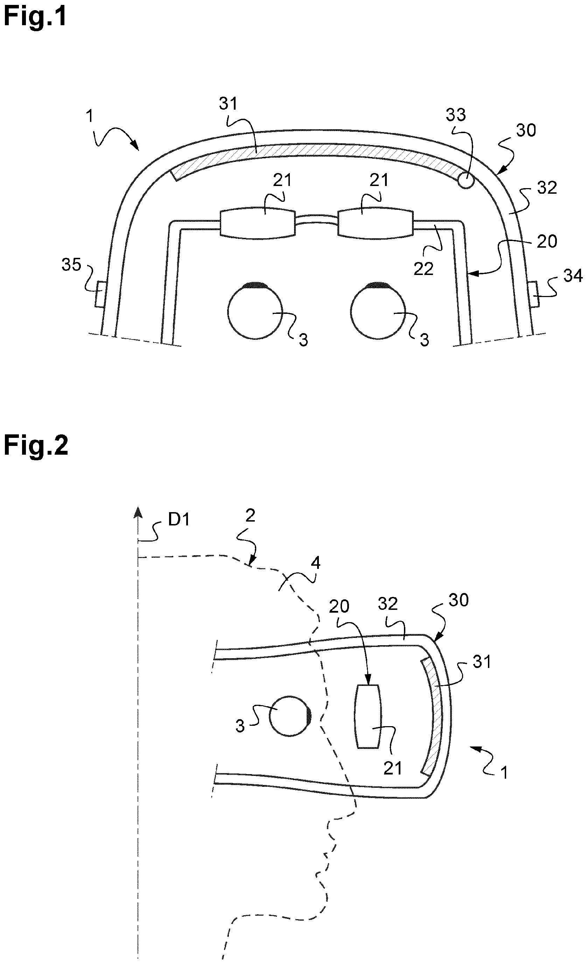

A system 1 for determining values of an optical feature of a corrective ophthalmic lens, that is to say a lens adapted to correct a vision defect, is illustrated in figures 1 and 2. The system 1 comprises in particular a trial optical corrective device 20 and an eyewear 30. The system 1 also comprises a processing unit.

The optical feature is relative to a visual correction provided to an individual 2. In the following, the optical feature characterizes both the trial optical corrective device 20 and the corrective ophthalmic lens, which are both intended to be worn in front of an eye 3 of the individual 2. This means that the trial optical corrective device 20 has the optical feature and that the corrective ophthalmic lens has the optical feature. Put differently, the optical feature is a characteristic of the trial optical corrective device 20 and of the corrective ophthalmic lens.

As described hereafter, the optical feature can take a plurality of values. The values of the optical feature might be different or equal for the trial optical corrective device 20 and for the corrective ophthalmic lens.

The optical feature comprises for instance a dioptric optical feature, i.e. an optical feature measured in diopters. The optical feature comprises for instance an orientation, i.e. an optical feature representing an angle, for example measured in degrees. The optical feature comprises for instance one of the following: a sphere power, a cylinder power, a cylinder axis, a spherical power addition, a prism power, a prism axis. The values of the optical features are then for instance expressed in diopters or in degrees.

The trial optical corrective device 20 is intended to be worn by the individual 2 in front of his eye 3 in order to test a given value of the optical feature, that is to say to subjectively assess or evaluate a correspondence between the given value and a need of the individual 2 for visual correction. The trial optical corrective device 20 thus provides a visual correction complying with the given value. Here, "complying with" means that the value of the optical feature of the trial optical corrective device 20 is equal to the given value. However, as a variation, "complying with" may mean that the value of the optical feature of the trial optical corrective device 20 is derived from the given value. For instance, the trial optical corrective device 20 may provide a visual correction complying with the given value in the sense that the value of the optical feature of the trial optical corrective device 20 plus a value of the optical feature of a specific lens of the eyewear 30 (as described later) is equal to the given value. For instance, the trial optical corrective device 20 may provide a visual correction complying with the given value in the sense that the value of the optical feature of the trial optical corrective device 20 is a rounded value of the given value (e.g. 1,3D rounded to 1,25D).

The trial optical corrective device 20 comprises at least one optically active part 21. The trial optical corrective device 20 may comprise two optically active parts 21 as illustrated in figure 1. The optically active parts 21 are respectively located in front of each eye 3. This allows testing a given value of the optical feature for one eye 3 and another given value for the other eye 3, the values being identical or different.

Only one optically active part 21, referred to as the optically active part 21, and only one eye 3, referred to as the eye 3, in front of which the optically active part 21 is to be arranged, are described hereafter.

The optically active part 21 is "active" in the sense that it corrects, or at least affects, the vision of the eye 3. The optically active part 21 comprises for instance one or more trial lenses each having a predetermined optical property. The predetermined optical property is here to a value of the optical feature to be tested. The optically active part 21 may also comprise an active lens having an adaptive optical property. The adaptive optical property is for instance an adjustable sphere power which can be electronically controlled. The active lens is for instance a polymer lens or a liquid lens whose shape can be electrically controlled, a liquid crystal (electroactive) lens, or phase-only spatial light modulators (SLMs), or any other suitable component. The optically active part 21 may also comprise a movable lens 22 such that a distance between the movable lens and the eye 3 is adaptable. The optically active part 21 may also comprise a light field display as described in WO2022/121640.

As described in detail hereafter, the value of optical feature of the trial optical corrective device 20 can be adapted, i.e. modified. This adaptation is for instance performed by swapping, the trial lens having a predetermined optical property to another trial lens having another predetermined optical property, or by adding the other trial lens in front of the trial lens. This adaptation can also be performed by modifying the adaptive optical property of the active lens or the distance with respect to the eye 3 of the movable lens.

The eyewear 30 is adapted to immerse the individual 2 in a virtual environment, more specifically in a visual virtual environment. Here, the eyewear 30 is more specifically a headset 30 adapted to be fastened on the head 4 of the individual 2. The headset 30 is designed to present content to the individual 2, including images and video. Images and video may be combined with audio via speakers or headphones. In the example illustrated in figures 1 and 2, the headset 30 is adapted for virtual reality, which means that the environment seen by the individual 2 is exclusively provided by the headset 30.

As a variation, the headset may be adapted for mixed reality, which means that the virtual environment viewed by the individual is an overlay or a merge of the physical environment surrounding the individual, which includes objects, people, or parts of the body of the individual, and of virtual elements provided by the headset.

The headset 30 is a head-mounted display adapted to be placed in front of the eye 3 of the individual 2. The headset 30 more specifically comprises a screen 31 and holdings means 32 holding the screen 31 in front of the eye 3 as represented in figures 1 and 2.

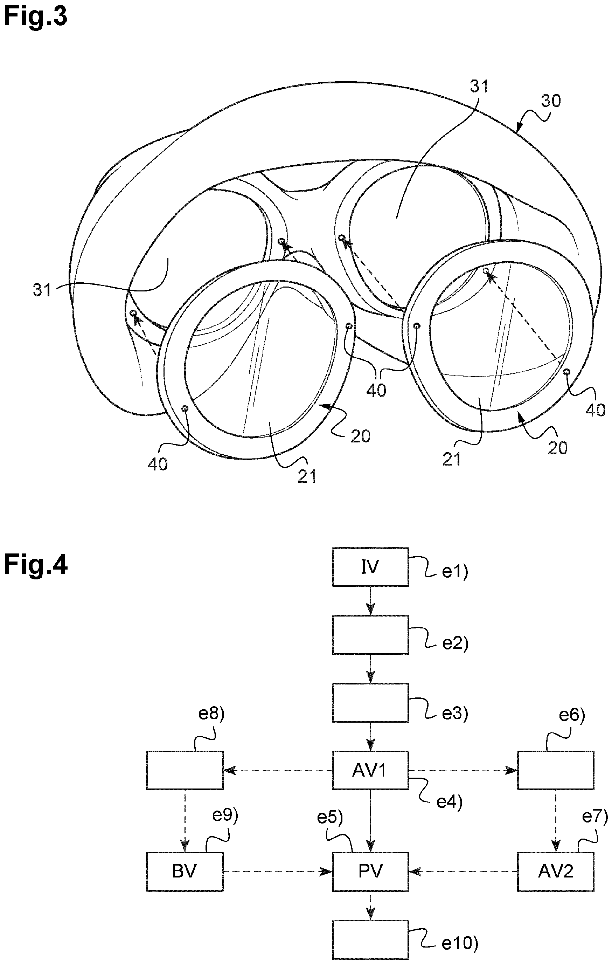

In a first embodiment, the trial optical corrective device 20 is mounted in the headset 30, i.e. attached to it. The trial optical corrective device 20 is for instance fixed to the headset 30 by magnets 40, as figure 3 shows it, or by clips. The trial optical corrective device 20 could also be embedded in the headset 30. In the first embodiment, the holding means 32 of the headset 30 also allows to maintain the trial optical corrective device 20, and more specifically the optically active part 21, in front of the eye 3.

In a second embodiment, the trial optical corrective device 20 comprises a frame 22 in which the optically active part 21 is mounted in, as represented in figure 1. The frame 22 is adapted to be placed on the head 4 of the individual 2, in particular so that the optically active part 21 is respectively located in front of the eye 3.

In this case as shown in figure 1, the headset 30 is adapted to be placed on the head 4 of the individual 2 when the individual 2 is wearing the trial optical corrective device 20 and especially the frame 22. The headset 30 is thus adapted to be placed in front of the trial optical corrective device 20. In particular, the holding means 32 is also adapted to the presence of the frame 22 on the head 4.

In all embodiments, the trial optical corrective device 20 is thus placed between the eye 3 and the headset, as illustrated in figures 1 and 2. The distance between the screen 31 and the eyes 3 is sufficient to arrange the trial optical corrective device 20 between the screen 31 and the eyes 3.

The screen 31 is for instance designed to display stereoscopic images, that is to say to display slightly different images to each eye 3 in order to build a three-dimensional scene for the individual 2. The virtual environment depth generated by the headset 30 is thus at least based on a convergence effect by mixing different images on each eye 3.

Preferentially, the headset 30 is also adapted to produce the virtual environment based on an accommodation effect (or focusing effect). This accommodation effect is for instance obtained by providing a different focus for each pixel of the screen 31, for instance by modifying the optical path between each pixel, or distinct group of pixels, and each eye 3. To do so, the headset 30 for instance comprises a varifocal element (not represented in figures 1 and 2), transmitting light from the screen 31 toward each eye 3, that is adapted to mechanically change its distance with the screen 31 or to change its shape, optical path, or refraction index. The optical properties of the headset 30 are for example described in document WO2022/121640.

With respect to its technical features, the headset 30 has in particular a pixel angle resolution lower than 1 minute, a projection distance comprised between 75 cm and 1,5 m, a field of view greater than 90 degrees, a display frame rate greater than 60Hz and a luminance greater than 25 cd/m². Those features allow controlling efficiently the illumination conditions when the individual is immersed in the virtual environment and also producing a high-quality virtual environment.

In addition, the headset 30 may comprise one or more specific lenses to immerse the individual 2 in the virtual environment. The one or more specific lenses of the headset 30 then face the optically active part 21 of the trial optical corrective device 20 such that the eye 3 views the images displayed by the screen 31 through both the optically active part 21 and the one or more specific lenses.

The trial optical corrective device 20, and in particular the optically active part 21, could also consist in, i.e. be formed by, the one or more specific lenses of the headset 30. In this case, the one or more specific lenses of the headset 30 also have the optical feature. The value of optical feature of the trial optical corrective device 20 could be adapted, i.e. modified, by swapping the one or more specific lenses.

As shown in figure 1, the headset 30 may also comprise a motion tracking system33, which for instance comprises a gyroscope, an accelerometer, or a magnetometer. The motion tracking system 33 allows to track motions of the head 4 of the individual 2. The motion tracking system 33 allows detecting movements of the head 4 with respect to at least one degree of freedom, for instance a rotation around the vertical direction D1 represented in figure 2. Preferentially, the motion tracking system 33 allows detecting movements of the head 4 with respect to six degrees of freedom, three in rotation and three in translation, such as to fully depict the movements of the head 4. The motion tracking system 33 is for instance mounted on the holding means 32 as represented in figure 1.

In order to track motions of the head 4 of the individual 2, the headset 30 may also comprise landmarks or markers. The landmarks or markers can then be detected by external cameras. The landmarks or markers may also be photodiodes that are detected by spinning lasers. In a general way, any triangulation or 3D scanning system that allow to determine the orientation of the headset 30 may be implemented.

As shown in figure 1, the headset 30 also comprises eye-tracking sensors 34 for example located next to the screen 31.

The virtual environment seen by the individual 2 when equipped with the headset 30 (i.e. the content displayed by the screen 31) depends on a position of the head 4, for instance with respect to a 3D-referential linked to the ground on which the individual 2 stand or to a chair on which he is seated. The virtual environment for instance scrolls from left to right when the individual 2 rotates his head 4 to the left with respect to the vertical direction D1.

Here, the headset 30 also comprises a control unit 35 allowing controlling the content presented to the individual 2, in particular to control the images displayed by the screen 31. The control unit 35 comprises one or more processors and one or more memories. The memory is a computer-readable storage medium which comprises instructions allowing to operate the headset 30 when implemented by the processor. The instructions are representative of the content presented to the individual 2. The control unit 35 also comprises an input interface for adding instructions on the memory or updating the instructions on the memory, for instance in order to provide new content or content personalized to the individual 2. The input interface for instance comprises an antenna adapted to establish a wireless communication with a computer or a USB port.

As a variation, the control unit may be a distinct computer separated from the headset and communicating with the headset via a graphical interface, wirelessly or by wire.

Therefore, the light of the screen 31, i.e. the content displayed to the individual 2 by the headset 30, is seen by the individual 2 through the trial optical corrective device 20, and more particularly through the optically active part 21.

The headset 30 is more particularly adapted, in the sense that the control unit 35 is programmed for it, to present visual tasks to the individual 2. The headset 30 more specifically has in memory a set of visual tasks that can be performed by the individual 2 in the virtual environment. The set of visual tasks is stored on the memory of the control unit 35. The set of visual tasks preferentially comprise a plurality of visual tasks. As a variation, the set of visual tasks comprises only one visual task.

As described below, the visual tasks are visual assignments, exercises, or activities that the individual 2 performs when he is immersed in the virtual environment. When performing the visual tasks, the individual 2 needs to move his eye 3 and may need to move his head 4 and even his body. In other words, the individual 2 is active when performing visual tasks in the sense that he performs actions.

The visual tasks are for instance representative of an activity that the individual 2 intends to perform when wearing the corrective ophthalmic lens.

The visual tasks are for instance representative of activities such as driving a car, biking, walking, using a digital device (computer, tablet, smartphone), watching TV, reading while seating, standing or walking, crossing a road, shopping in a supermarket or in a library, etc. The visual tasks for instance reproduce reading on supports such as books, smartphones, or computer screens. Thanks to the virtual environment, different conditions can be interpreted, for example day or night driving, different character size for reading, or walking indoors or outdoors.

Preferentially, the virtual environment reproduces an environment known by the individual 2, e.g. his home or his workplace, such that the individual 2 is able to virtually read on his personal laptop or a model close to it.

The visual tasks may also take into account a lifestyle of the individual 2, an environment in which the corrective ophthalmic lens is intended to be worn or preferences of the individual 2. For instance, when the individual 2 needs the corrective ophthalmic lens mainly indoors, the visual tasks mainly reproduce indoor environments. The individual 2 may need the corrective ophthalmic lens mainly for static activities, the visual tasks may then include seating, reading, or mainly for dynamic activities, the visual tasks may then include walking.

The visual tasks may also take into account personal features of the individual 2 such as an age individual 2 or a size of the individual 2. For instance, the size of the characters of a reading visual task may depend on the age of the individual: the older the individual 2 is, the bigger the characters are. For instance, the younger the individual 2 is, the greater the speed of a visual task is, for instance when it includes gaming.

The visual tasks may also include games to be played by the individual 2.

In a general manner, the visual tasks mimic different real-life conditions. The individual 2 is thus advantageously placed in a natural posture when performing the visual tasks, which means that the physical posture of the individual 2 during the visual tasks is close to the physical posture he will adopt when using the corrective ophthalmic lens. Indeed, the individual 2 for instance walks in a more natural way when wearing the headset 30 than when he feels spatially constrained in the refraction room. Still in example, when the visual tasks reproduce a driving session, the individual 2 holds and moves his head 4 as he would naturally do when driving.

Preferentially, the visual tasks, i.e. virtual environment in which the individual 2 is intended to be immersed, are personalized or customized for the individual 2. Consequently, the posture adopted by the individual during the visual tasks is even more natural. For instance, thanks to the virtual environment, the individual 2 reproduces a posture corresponding to its real-life posture when reading on his laptop.

To do so, information from smart glasses previously used by the individual 2 may be taken into account. This information for example comprises working distances, that is to say usual distances to objects seen by the individual 2, or light conditions which can be acquired and stored by the smart glasses.

The virtual environment can also be personalized or customized by taking into account instructions of the individual 2, such as a description of his place or of his habits, or complaints of the individual 2 with respect to previous visual tasks he performed. These information, instructions or complaints can be transmitted to the control unit 35 by means of the input interface.

As a variation, when the headset is adapted for mixed reality, the real scene can be seen through the lenses of the headset while virtual elements can be superimposed in the visual field of the individual. In this case, the real scene is used as a visual context and virtual elements are used to personalize the virtual environment.

Figure 4 illustrates a method for determining a personalized value PV of the optical feature. The method is here implemented by the system 1. The method could be implemented by any suitable system. The method is implemented for at least one eye 3 of the individual 2.

As shown in figure 4, the method comprises the main steps of:
e1) determining an initial value IV of the optical feature based on a visual test performed by the individual 2;
e2) equipping the individual 2 with the trial optical corrective device 20, the trial optical corrective device 20 providing a correction complying with the initial value IV, and with the headset 30;
e3) a step wherein the individual 2 performs a visual task, hereafter called the first visual task, in the virtual environment;
e4) determining an adjustment value AV1 of the optical feature based on a result of the first visual task;
e5) calculating the personalized value PV based on the initial value IV and the adjustment value AV1.

The method thus starts by step e1) of determining the initial value IV based on the visual test. The visual test allows determining a vision correction for the eye 3.

The visual test is preferentially a subjective test. Standard subjective tests that can be implemented in the context of the disclose are for instance described in EP3424009.

The subjective test is associated with an optical situation provided to the individual 2. The subjective test comprises an assessment of the vision of the individual 2, by the individual 2 itself, when placed in the optical situation.

The subjective test is performed under the supervision of an eye-care professional such as an optometrist. The subjective test is for instance performed by means of a phoropter.

The outcome of the subjective test comprises an interpretation, by the eye-care professional, of the assessment of the individual 2. Based on this interpretation, the eye-care professional determines the initial value IV. In other words, the eye-care professional determines the initial value IV based on a subjective answer of the individual 2 to the subjective test.

By way of example, the optical situation can comprise a comparison between two conditions. The two conditions are for instance two targets of different size, two identical targets placed in a green environment for one and in a red environment for the other, or a same target seen through two distinct trial lenses which have for example two distinct sphere power or two distinct cylinder axis. The subjective test comprises assessing which one of the two conditions provides higher visual performance according to the individual perception. To obtain this information, depending on the subjective test performed, the eye-care professional may for example ask, *"in which situation do you better* see *the target"* or *"which target do you better see"*.

Usually, the same optical situation is iterated with different correction provided to the individual, for instance -0.25D or +0.25 for sphere power between two iterations of the optical situation. The eye-care professional may for example ask *"do you now see better the visual target with this correction".* This interpretation then takes into account how the subjective answers of the individual 2 evolves between the iterations.

The subjective test may comprise: a duochrome test, a cross cylinder test, an acuity test without accommodation relaxation, an acuity test with accommodation relaxation, a bi-ocular balance test.

By way of example, it can be determined during the subjective test that the eye 3 needs an additional 2 D (diopters) for sphere power. In this example, it means that the optical feature is the sphere power and that the initial value IV is 2 D.

The visual test may also be an objective test, such as an objective refraction test. The objective test is for instance performed by means of an auto-refractor.

As a variation, the determination of the initial value comprises collecting data representative of a vision correction, for instance from a storage device such as a distant server or a USB drive. These data are for instance resulting from a test performed by the individual by himself, for example at home. The initial value may then be deduced from these data, for instance by the eye-care professional.

In step e2), the trial optical corrective device 20 and the headset 30 are provided to the individual 2.

Step e2) comprises setting the value of the optical feature of the trial optical corrective device 20 to the initial value IV, i.e. such that it complies with the initial value IV.

The trial optical corrective device 20 thus provides to the eye 3 the vision correction determined during the visual test. In other words, the trial optical corrective device 20 provides a correction complying with the initial value IV.

For instance, when the initial value is 2 D for sphere power, the trial optical corrective device 20 may comprise a trial lens having a predetermined sphere power of 2 D as the optically active part 21. Alternatively, the trial optical corrective device 20 may also comprise an active lens whose shape is determined such as to provide a sphere power of 2 D.

As a variation, the value of the optical feature of the trial optical corrective device may be derived from the initial value by taking into account the difference in vertex distance from the eye between an eye-lens distance used in step e1) and the distance between the eye and the optically active part in step e2). The value of the optical feature of the trial optical corrective device may also take into account a combination with the one or more specific lens of the headset when distinct form the optically active part.

In the first embodiment, step e2) comprises attaching the trial optical corrective device 20 to the headset 30. Then, since the trial optical corrective device 20 is mounted in the headset 30, placing the headset 30 on the head of the individual 2 also places the trial optical corrective device 20 on the head of the individual 2.

In the second embodiment, wherein the trial optical corrective device 20 comprises the frame 22, step e2) comprises a first substep of arranging the trial optical corrective device 20 in front of the eye 3, i.e. of placing the trial optical corrective device 20 on the head 4 of the individual 2. This arrangement may be performed before or after setting the value of the optical feature of the trial optical corrective device 20 to the initial value IV. Step e2) then comprises a second substep of providing the headset 30 to the individual 2, who is already wearing the trial optical corrective device 20.

In all embodiments, at the end of step e2), the headset 30 is thus placed on the head 4 of the individual 2 such that :
- the optically active part 21 is located in front of the eye 3,
- the optically active part 21 has an optical feature whose value complies with, for instance is equal to, the initial value IV,
- the individual 2 views the content presented by the headset 30, i.e. the virtual environment, through the trial optical corrective device 20.

In other words, the optically active part 21 is interposed between the eye 3 and the screen 31 of the headset 30.,

In step e3), the individual 2 performs the first visual task in the virtual environment. The first visual task belongs to the set of visual tasks previously described.

The visual task is performed by the individual wearing the trial optical corrective device 20. The individual 2 is more specifically wearing the trial optical corrective device 20 when it is providing a correction complying with the initial value IV.

The method then comprises step e4) of determining the adjustment value AV1, hereafter referred to as the first adjustment value AV1, based on a result of the first visual task. The result of the first visual task may be collected during the first visual task or at the end of the first visual task.

The result of the first visual task may for instance be a feedback, that is to say a comment, formulated by the individual 2 about the first visual task. The feedback is a subjective assessment or evaluation of the first visual task by the individual 2. In other words, the feedback is a perception of the first visual task by the individual 2 or a reaction of the individual 2 to the first visual task.

The feedback may be the expression of a visual discomfort that the individual 2 feels in the virtual environment during the first visual task. Such a discomfort is for instance due to a visual fatigue, or a blurred vision experienced during the first visual task. On the contrary, the feedback may be the expression of a visual comfort that the individual 2 feels in the virtual environment. Such a comfort is representative of the fact that the correction provided by the trial optical corrective device 20 is adapted for the individual 2. The feedback may be a rating of vision quality, in global or for a particular working distance.

The eye-care professional is then able to determine, based on the feedback, the first adjustment value AV1. To do so, the eye-care professional may use his practice, his experience and knowledge as well as charts or value tables.

By way of example, when the individual 2 expresses a discomfort due to a blurred virtual environment, the eye-care professional may determine that the individual 2 needs less (or more negative)sphere power than what is currently provided by the trial optical corrective device 20. The first adjustment value AV1 is then for instance determined as -0.25 D (minus 0.25 diopters) for sphere power. Still in example, when the individual 2 expresses visual fatigue, the eye-care professional may determine that the individual 2 needs more (or less negative) sphere power than what is provided by the trial optical corrective device 20. The first adjustment value AV1 is then for instance determined as +0.25 D (plus 0.25 diopters) for sphere power. The other way around, when the individual 2 feels comfortable during the first visual task, the first adjustment value AV1 is for instance determined as null.

Still in example, the first adjustment value AV1 may be determined by comparing two values of the optical feature during the visual task, i.e. a comparison between two possible adjustments. The two possible adjustments are for instance with and without +0.25 D or -0.25 D with respect to the initial value IV or with -0.25 D and +0.25 D with respect to the initial value IV.

The result of the first visual task may also be a user input performed by the individual 2. The user input is for instance acquired by means of a user interface. The user input for instance allows the individual 2 to express that he was comfortable or uncomfortable during the first visual task. The user interface may then comprise two buttons, one for expressing comfort and one for expressing discomfort. Preferentially, the interface allows the individual 2 to rate his comfort and discomfort, for instance on a scale of one to five or one to ten. The first adjustment value AV1 is then for instance determined by a computer system, i.e. computed based on the user input and according to a predetermined adjustment rule, in particular when it is determined based on a comparison between two possible adjustments. The first adjustment value AV1 may also be determined by the eye-care professional based on the user input.

The result of the first visual task may also be an objective assessment comprising at least one of the following: a measurement of a posture of the individual 2, a measurement of a head of gaze direction, a performance or a success rate of the individual 2 performing the first visual task. This measurement of a gaze direction is for instance performed by means of the eye-tracking sensor 34 of the headset 30. The measurement of a posture of the individual 2 is for instance performed by the motion tracking system 3. The measurement of a posture may also take into account hands, feet or bust movements. The first adjustment value AV1 is then for instance determined by a computer system, i.e. computed based on the user input and according to a predetermined adjustment rule.

The first adjustment value AV1 is then for instance determined by comparing the postures for the two possible adjustments above mentioned. The first adjustment value AV1 may be the one of the two possible adjustments providing the posture the closer to the natural posture of the individual 2. The natural posture may be determined from previous measurements, from a reference visual task or from ergonomic data that define the best posture to have for a given visual task.

The first adjustment value AV1 may be a multiple of a predetermined basic value of the optical feature. Such a predetermined basic value is for instance 0.25 when the optical feature is expressed in diopters or 5 when the optical feature is expressed in degrees. The first adjustment value AV1 may also be any real number. In a general manner, the first adjustment value AV1 may be positive, negative, or null.

In step e5), the personalized value PV of the optical feature is calculated based on the initial value IV and the first adjustment value AV1. The personalized value PV is more specifically calculated at least based on the initial value IV and the first adjustment value AV1. Indeed, as described below, other values may be taken into account to calculate the personalized value PV.

The calculation of the personalized value PV is here performed by the processing unit which for instance comprises a calculator or a computer system. The processing unit is more specifically programmed to calculate the personalized value PV.

Preferentially, the personalized value PV is calculated as the sum of the initial value IV and of the first adjustment value AV1.

Thus, on the one hand, when the first adjustment value AV1 is determined as null, the personalized value PV is equal to the initial value IV. Indeed, the first adjustment value AV1 being null means that the correction provided by the trial optical corrective device 20, i.e. the initial value IV, is adapted to the individual 2. In other words, the first adjustment value AV1 being null means the individual 2 deems the correction provided by the trial optical corrective device 20 to be well-adapted when testing it in the virtual environment. In this case, the first visual task of step e3) thus allows to validate that initial value IV is well customized for the use of the corrective ophthalmic lens by the individual 2.

On the other hand, when the first adjustment value AV1 is determined as different from zero, the personalized value PV is then different from the initial value IV. Indeed, the first adjustment value AV1 being non-null means that the correction provided by the trial optical corrective device 20, i.e. the initial value IV, is not well-adapted for the individual 2. In other words, the first adjustment value AV1 being non-null means the individual 2 deems the correction provided by the trial optical corrective device 20 to be unadapted when testing it in the virtual environment. In this case, the first visual task of step e3) thus allows to determine a value distinct from the initial value IV, namely the personalized value PV, that is more customized for the use of the corrective ophthalmic lens by the individual 2 than the initial value IV.

As a variation, the personalized value may be calculated as a rounded sum of the initial value and of the adjustment value. This sum is for instance rounded to a multiple of the predetermined basic value above-mentioned. The personalized value may also be calculated as the closest value, among a predetermined list of values, from the sum of the initial value and of the adjustment value. This can make the manufacturing and/or the ordering of the corrective ophthalmic lens less costly. For instance, when the optical feature is in diopters, the sum of the initial value and of the adjustment value may be automatically rounded by the processing unit to the nearest quarter dioptre (e.g. 1,3D is rounded to 1,25D), or eighth dioptre (e.g. 1,15D is rounded to 1,125D) to obtain the personalized value.

Still in variation, the personalized value may be based on the sum of the initial value and of the adjustment value, the sum being further modified based on needs or personal features of the individual, such as his age.

Steps e2) to e5) therefore correspond to a validation phase of the initial value IV determined based on the visual test in step e1). This means that the initial value IV is tested to determine whether or not it suits the individual. Thanks to the virtual environment, the individual is in a natural posture when testing the initial value IV. Moreover, the first visual task is preferentially personalized to the individual such that the initial value IV is tested in situations near to real-life situations relatively to the individual 2. This validation phase results in the personalized value PV of the optical feature of the corrective ophthalmic lens, that is to say a value that accurately matches the vision correction needs of the individual 2.

Advantageously, the individual 2 may perform several visual tasks during step e3). Those several visual tasks belong to the set of visual tasks. This allows in particular testing the trial optical corrective device 20 in different conditions, for instance different illumination conditions (day, night, etc.) or for different activities (driving, reading, etc.).

In a remarkable way, the results of the several visual tasks may be weighted for determining the adjustments value AV. The results are for instance weighted based on a lifestyle or preferences of the individual 2. For instance, when the individual 2 intends to use the corrective ophthalmic lens mainly for driving, the results of the several visual tasks that reproduce driving experiences may have greater weights.

The first adjustment value AV1 may also be determined as a compromise between the results of the several visual tasks, e.g. an average or a median of these results. This compromise may be chosen by the individual 2 or estimated by the eye-care professional according to comments of the individual 2.

In a remarkable way, the method may comprise an iteration of visual tasks, the value of the optical feature of the trial optical corrective device 20 being modified between two successive iterations. The idea is to successively test the result of the previous visual task in order to further customize the personalized value PV.

To this end, the method further comprises a step e6) and a step e7) that are implemented in a raw. As figure 4 shows it, step e6) and e7) are performed after step e4) and before step e5).

Step e6) comprises adapting the trial optical corrective device 20 such that the trial optical corrective device 20 provides a correction complying a sum of the initial value IV and the first adjustment value AV1. In other words, the value of the optical feature of the trial optical corrective device 20 is modified such that it complies with, here is equal to, the sum of the initial value IV and the first adjustment value AV1. This modification may be performed as previously described, for example by adding or swapping trial lenses or changing the shape of an active lens.

The adaptation of the trial optical corrective device 20 may be performed while the individual is equipped with the headset 30. Alternatively, the individual may remove the headset 30 for the modification of the trial optical corrective device 20 and then put the headset 30 back on. Both the trial optical corrective device 20 and the headset 30 may also be removed from the head 4 of the individual 2, then the trial optical corrective device 20 may be modified, and finally the trial optical corrective device 20 and the headset 30 may be put back on.

In any case, at the end of step e6), the individual 2 is equipped with the headset 30 and the optically active part 21 is located in front of the eye 3.

The method then continues with step e7) comprising determining another adjustment value AV2, hereafter referred to as the second adjustment value AV2, of the optical feature based on another visual task, hereafter referred to as the second visual task, performed by the individual 2. The second visual task also belongs to the set of visual tasks. Therefore, the second visual task also mimics different real-life conditions and is preferentially personalized or customized to the individual 2.

In a similar way to step e4), the second adjustment value AV2 is determined based on a result of the second visual task. The result of the second visual task may be a feedback, a user input or an objective assessment as above-described. The second adjustment value AV2 may be a multiple of a predetermined basic value of the optical feature, for instance 0.25 when the optical feature is expressed in diopters or 5 when the optical feature is expressed in degrees. The second adjustment value AV2 may also be any real number. In a general manner, the second adjustment value AV2 may be positive, negative, or null.

The second visual task performed in step e7) may be the same visual task as the first visual task performed in step e3). This allows fine-tuning the personalized value PV for a specific activity that the individual 2 intends to perform when wearing the corrective ophthalmic lens and that is reproduced by the virtual environment.

The second visual task performed in step e7) may be different from the first visual task performed in step e3). This allows to determine the personalized value PV so that the corrective ophthalmic lens is efficient in a plurality of real-life situations.

When performing step e6) and step e7), the calculation of the personalized value PV in step e5) is then also based on the second adjustment value AV2.

Preferentially, the processing unit calculates the personalized value PV as the sum of the initial value IV, and a combination of the first adjustment value AV1 and of the second adjustment value AV2. The personalized value PV is for instance the sum of the initial value IV, the first adjustment value AV1 and the second adjustment value AV2

When the second adjustment value AV2 is determined as null and when the second visual task is the same as the first visual task, the personalized value PV is equal to the initial value IV plus the first adjustment value AV1. Indeed, the second adjustment value AV2 being null means that the correction provided by the trial optical corrective device 20 during step e7) is adapted to the individual 2. In other words, the second adjustment value AV2 being null means the individual 2 deems the correction provided by the trial optical corrective device 20 to be well-adapted when it is equal to the initial value IV plus the first adjustment value AV1. In this case, the second visual task of step e7) thus allows to validate that sum of the initial value IV and the first adjustment value AV1 is a value well customized for the use of the corrective ophthalmic lens by the individual 2.

On the other hand, when the second adjustment value AV2 is determined as different from zero and when the second visual task is the same as the first visual task, the personalized value PV is then different from the initial value IV plus the first adjustment value AV1. Indeed, the second adjustment value AV2 being non-null means that the correction provided by the trial optical corrective device 20 during step e7) is not well-adapted for the individual 2. In other words, the second adjustment value AV2 being non-null means the individual 2 deems the correction provided by the trial optical corrective device 20 to be unadapted when it is equal to the sum of the initial value IV and of the first adjustment value AV1. In this case, the visual task of step e7) thus allows to determine another value, namely the personalized value PV, that is more customized for the use of the corrective ophthalmic lens by the individual 2 than the sum of the initial value IV and of the first adjustment value AV1.

As a variation, the personalized value may be calculated as a rounded sum of the initial value, and a combination of the adjustment value and of the second adjustment value (e.g. a sum of those three values). The personalized value may also be calculated as the closest value, among a predetermined list of values, from the sum of the initial value, of the adjustment value and of the second adjustment value. Still in variation, the personalized value may be based on the sum of the initial value, of the adjustment value and of the second adjustment value, said sum being further modified based on needs or personal features of the individual.

Step e6) and step e7) may be repeated for instance such as to determine a third adjustment value and a fourth adjustment value and the personalized value may be calculated as the sum of the initial value with a combination of each adjustment value. Step e6) and e7) are for instance repeated until the individual is satisfied with the correction provided by the trial optical corrective device during a visual task.

In a remarkable way, the method may comprise the determination of a potential bias due to the virtual environment. The idea is to determine a potential difference between a vision correction need of the individual 2 in the physical environment and a vision correction need of the individual 2 in the virtual environment.

To this end, as figure 5 shows it, the method further comprises a step e8) wherein the individual 2 performs a bias visual task 2 in the virtual environment. The bias visual task is performed when the individual is equipped with the headset 30 and the trial optical corrective device 20.

As illustrated in figure 5, step e8) is performed after step e4), the value of the optical feature of the trial optical corrective device 20 thus complies with the initial value IV.

The bias visual task is specifically designed to reproduce the visual test performed is step e1) for determining the initial value IV. In other words, the virtual environment mimics the optical situation provided to the individual 2 during the visual test in step e1). It means for instance that the bias visual task is designed to reproduce the comparison between the two conditions of step e1). The bias visual task is thus a virtual visual test equivalent, in the virtual environment, to the physical visual test of step e1).

For instance, the content presented to the individual 2 during the bias visual task comprises visual targets identical to those used during the visual test, that is to say visual targets having identical shapes and identical eye-target distances than those used during the visual test. By way of example, when the visual test is a duochrome test, the bias visual task also comprises display targets overlaid on a red and on a green background.

The method then continues with a step e9) of determining a bias value BV of the optical feature based on the bias visual task, i.e. on a result of the bias visual task.

The result of the bias visual task may be that the individual 2 feels no need for correction when viewing the optical situation provided to him. This is for instance the case when the individual 2 feels visually comfortable during the bias visual task, for instance because the visual targets are not blurred or because he perceives no difference when comparing two conditions. This result for instance happens when the individual views the targets overlaid on a red and on a green background with the same clearness. In a general way, it corresponds to a case wherein the answer of the virtual visual test given by the individual 2 is interpreted by the eye-care professional as a correct vision of the individual 2, i.e. without a need for correction.

Put differently, there is no difference between a vision correction need of the individual 2 in the physical environment and a vision correction need of the individual 2 in the virtual environment. Indeed, during the virtual visual test, the individual 2 is wearing the trial optical corrective device 20 which is providing a correction complying with the initial value IV. The correction with the initial value IV is efficient in the virtual environment. The bias value BV of the optical feature is then determined as null, i.e. equal to zero.

The result of the bias visual task may also be that the individual 2 feels a need for correction when viewing the optical situation provided to him in the virtual environment. This is for instance the case when the individual 2 feels a visual comfort during the bias visual task, for instance because the visual targets are blurred or because he perceives differences when comparing two conditions. This happens when the individual views the targets overlaid on a red and on a green background with different neatness. In a general way, it corresponds to a case wherein the answer of the virtual visual test given by the individual 2 is interpretated by the eye-care professional as an incorrect vision of the individual 2, i.e. with a need for correction.

Put differently, there is a difference between a vision correction need of the individual 2 in the physical environment and a vision correction need of the individual 2 in the virtual environment. Indeed, during the virtual visual test, the individual 2 is wearing the trial optical corrective device 20 which is providing a correction complying with the initial value IV. The correction complying with the initial value IV is not efficient in the virtual environment. The bias value BV of the optical feature is then determined as different from zero. The eye-care professional is then able to determine, based on the answers of the individual 2, the bias value BV based on his practice or based on predetermined procedures. The bias value BV determined during the virtual visual test is determined according to the same procedure as the initial value IV during the physical visual test of step e1).

By way of example, when the individual 2 perceives a target to be sharper on a reg background than on a green background during the virtual visual test, the bias value BV is for instance determined as +0.25 D for sphere power.

The bias value BV may be a multiple of a predetermined basic value of the optical feature, for instance 0.25 when the optical feature is expressed in diopters or 5 when the optical feature is expressed in degrees. The bias value BV may also be any real number. In a general manner, the bias value BV may be positive, negative, or null.

The calculation of the personalized value PV in step e5) is then also based on the bias value BV. The personalized value is more specifically corrected to account for the bias value BV. This means that the personalized value PV is calculated as the sum of the initial value IV and of the first adjustment value AV1, from which the bias value BV is subtracted. Remarkably, by subtracting the bias value BV which can be null or different from zero, any potential bias due to the virtual environment is then taken into account when calculating the personalized value PV.

When the method comprises both the determination of a bias and an iteration of visual tasks, step e8) and e9) are performed before step e6) and e7). The personalized value PV may be calculated as the sum of the initial value with each adjustment value, from which the bias value is subtracted.

In a remarkable way, the method further comprises a step e10) of demonstrating the personalized value to the individual 2 in the virtual environment.

Step e10) thus comprises providing a final optical device in front of the eye 3 of the individual 2. The final optical device provides a correction complying with the personalized value PV. In other words, this means that the optical feature also characterizes the final optical device and that the value of the optical feature of the final optical device complies with, here is equal to, the personalized value PV.

The final optical device is a specific device which for instance comprises a frame in which ophthalmic lenses are mounted. The final optical device allows testing a specific design or type of ophthalmic lens such as unifocal lenses, progressive lenses, bifocal lenses, or lenses adapted for myopia progression control.

The final optical device to be validated may also comprise a real part representing the personalized value PV and a virtual part, i.e. a part simulated in the virtual environment, representing the design variation from the personalized value (for instance the lens design).

For instance, when the corrective ophthalmic lens is a progressive lens, the headset and the trial optical corrective device are not physically providing a display with different eye-object distances. The headset then simulates different eye-object distances by blurring the virtual environment accordingly.

Step e10) then comprises a validation of the final optical device based on a validation visual task performed by the individual 2 in the virtual environment. The validation visual task belongs to the set of visual tasks. When performing the validation visual task, the individual 2 is thus wearing the final optical device and the headset 30, the final optical device being interposed between eye 3 and the headset such that the individual views the virtual environment through the final optical device.

The validation of the final optical device may comprise a decision on the specific design or type of ophthalmic lens tested thanks to the final optical device. When the feedback from the individual resulting from the validation visual task is satisfactory, the specific design or type of ophthalmic lens tested may be provided to the individuals as the corrective ophthalmic lens. When the feedback from the individual resulting from the validation visual task is not satisfactory, another specific design or type of ophthalmic lens may be tested in another iteration of step e10).

The method may also further comprise a step of designing and/or manufacturing the corrective ophthalmic lens such that the value of the optical feature of the corrective ophthalmic lens is equal to the personalized value PV.

The designing and/or manufacturing of the corrective ophthalmic lens may be performed on site by the eye-care professional. It may also be performed by a manufacturing-lab. In this case, the corrective ophthalmic lens is for instance ordered electronically by sending an electronic order from an optician computer system to a manufacturing-lab computer system. The order to the manufacturing-lab includes the personalized value PV and may also include the type and/or the design of the corrective ophthalmic lens such as unifocal lenses, progressive lenses, or bifocal lenses.

The method may further comprise a step selecting or collecting, in a stock of prefabricated lenses, the corrective ophthalmic lens such that the value of the optical feature of the corrective ophthalmic lens is equal to the personalized value PV.

The present invention is by no means limited to the embodiments described and shown, but the person skilled in the art will know how to make any variant in accordance with the invention.

For instance, the method is described above with respect to one of the eyes of the individual. However, the method can advantageously be implemented for both eyes in parallel, which allows determining a first personalized value for a corrective ophthalmic lens for the left eye and a second personalized value for another corrective ophthalmic lens for the right eye. When, the visual test is performed for both eyes, the trial optical corrective device comprises two optically active parts such as to test a first initial value with respect to the left eye at the same time as a second initial value with respect to the right eye. The adjustment values are often identical for both eyes, but when the initial values are not, the personalized values are distinct. In particular, the method could be implemented for both eyes when the visual test of step e1) is binocular. This allows adjusting the global visual comfort of the individual, for instance by determining a personalized sphere power difference between the two eyes.

## Claims

1. Method for determining a personalized value (PV) of an optical feature of a corrective ophthalmic lens which is intended to be worn in front of an eye (3) of an individual (2) for correcting his vision, the method comprising the steps of:
- determining an initial value (IV) of the optical feature based on a visual test performed by the individual (2);
- equipping the individual (2) with a trial optical corrective device (20) in front of the eye (3) of the individual (2), the trial optical corrective device (20) providing a correction complying with the initial value (IV) of said optical feature and with an eyewear (30), the trial optical corrective device (20) being placed between the eye (3) and the eyewear (30), the eyewear (30) being adapted to immerse the individual (2) in a virtual environment;
- determining an adjustment value (V1) of said optical feature based on a visual task performed by the individual (2) in the virtual environment;
- calculating the personalized value (PV) of said optical feature based at least on the initial value (IV) and the adjustment value (V1).

2. Method according to claim 1, wherein the trial optical corrective device (20) comprises means adapted to mount the trial optical corrective device (20) in the eyewear (30), or a frame (21) adapted to be placed on the head (4) of the individual (2) before placing the eyewear (30) on head (4) of the individual (2).

3. Method according to claim 2, wherein the adjustment value (V1) is determined based on a subjective assessment of the visual task by the individual (2) or on an objective assessment comprising at least one of the following: a measurement of a posture of the individual (2), a measurement of a head or gaze direction, a performance or a success rate of the individual (2) performing the visual task.

4. Method according to any one of claims 1 to 3, wherein the individual (2) is placed in a natural posture when performing the visual task.

5. Method according to any one of claims 1 to 4, wherein the visual task is representative of at least one of:
- an activity that the individual (2) intents to perform when wearing the corrective ophthalmic lens;
- an environment known by the individual (2);
- a lifestyle of the individual (2);
- an age individual (2);
- an environment in which the ophthalmic lens is intended to be worn;
- preferences of the individual (2).

6. Method according to any one of claims 1 to 5, wherein the virtual environment seen by the individual (2) when equipped with the eyewear (30) depends on a position of the head of the individual (2).

7. Method according to any one of claims 1 to 6, wherein the virtual environment is generated by virtual reality or mixed reality.

8. Method according to any one of claims 1 to 7, wherein determining the adjustment value (V1) of said optical feature is based on a plurality of visual tasks performed by the individual (2) in the virtual environment.

9. Method according to any one of claims 1 to 8 wherein the eyewear (30) has at least one of the following parameters: a pixel angle resolution smaller than 1 minute, a projection distance comprised between 75 cm and 1,5 m, a field of view greater than 90 degrees, a display frame rate greater than 60Hz, a luminance greater than 25cd/m².

10. Method according to any one of claims 1 to 9, further comprising a step of adapting the trial optical corrective device (20) such that the trial optical corrective device (20) provides a correction complying a sum of the initial value (IV) and the adjustment value (V1) and determining another adjustment value (V2) of said optical feature based on another visual task performed by the individual (2) in the virtual environment, and wherein calculating the personalized value (PV) of said optical feature is also based on said other adjustment value (V2).

11. Method according to any one of claims 1 to 10, further comprising a step of determining a bias value of the optical feature based on a bias visual task performed by the individual (2) in the virtual environment, said bias visual task being designed such that it reproduces the visual test performed for determining the initial value (IV), and wherein the personalized value (PV) is corrected to account for the bias value.

12. Method according to any one of claims 1 to 11, further comprising a step of placing a final optical device in front of the eye (3) of the individual (2), the final optical device providing a correction complying with the personalized value (PV), and of validating the final optical device based on a validation visual task performed by the individual (2) in the virtual environment.

13. Method according to any one of claims 1 to 12, wherein the trial optical corrective device (20) comprises at least one of the following:
- a trial lens having at least one predetermined optical property;
- an active lens having at least one adaptive optical property;
- a light field display;
- a movable lens mounted in a frame such that the distance between the movable ophthalmic lens and the eye (3) is adaptable.

14. Method according to any one of claims 1 to 13, the method further comprising a step of manufacturing, ordering, or selecting the ophthalmic lens such that the optical feature of the ophthalmic lens is equal to personalized value (PV) or to a rounded value determined by rounding the personalized value (PV) to the closest or the second closest multiple of a basic value.

15. System for determining a personalized value (PV) of an optical feature of a corrective ophthalmic lens which is intended to be worn in front of an eye (3) of an individual (2) for correcting his vision, the system comprising:
- a trial optical corrective device (20) adapted to be place in front of an eye (3) of an individual (2), the trial optical corrective device (20) providing a correction to an individual (2) complying with an initial value (IV) of an optical feature;
- an eyewear (30) adapted to be placed in front of the trial optical corrective device (20) worn by the individual (2) and adapted to immerse the individual (2) in a virtual environment, the eyewear (30) being designed to present, in the virtual environment, a visual task to the individual (2) adapted to determine an adjustment value (V1);
- a processing unit programmed calculate a personalized value (PV) of said optical feature based at least on the initial value (IV) and the adjustment value (V1).
